# EUROPÄISCHE PATENTSCHRIFT

(11) **EP 3 815 653 B1**
(45) Veröffentlichungstag und Bekanntmachung des Hinweises auf die Patenterteilung: **06.03.2024**
(21) Anmeldenummer: 20205614.9
(22) Anmeldetag: 04.11.2020
(51) Int. Cl.: A61F 5/058

(54) **NACKENSTÜTZE**
NECK SUPPORT
APPUI-TÊTE

(30) Priorität: 04.11.2019 DE 102019216940
(43) Veröffentlichungstag der Anmeldung: 05.05.2021
(73) Patentinhaber: Fraunhofer-Gesellschaft zur Förderung der angewandten Forschung e.V., 80686 München (DE)
(72) Erfinder: Schwarz, Dr. Oliver, 70569 Stuttgart (DE); Bölke, Nico, 70569 Stuttgart (DE)
(74) Vertreter: Pfenning, Meinig & Partner mbB

(56) Entgegenhaltungen:
- DE-A1-102011 018 272
- DE-B4- 10 196 653
- US-A- 3 359 976
- US-A1- 2010 298 748

## Beschreibung

Die Erfindung betrifft eine Nackenstütze mit einer Körperanbindung, die am Oberkörper einer Person befestigbar ist, einer Kopfstütze und einer Haltevorrichtung, über welche die Kopfstütze an der Körperanbindung angeordnet ist, wobei die Kopfstütze biegeelastische Schenkel aufweist, die durch eine Mehrzahl an Querstreben miteinander verbunden sind.

Bei längeren Überkopfarbeiten stellen sich Nackenstarre und Verspannungen sowie Verspannungskopfschmerzen ein, weil die Muskulatur nicht an diese ungewöhnliche Kopfhaltung angepasst ist. Eine Nackenspitze hält den Kopf so, dass die Nackenmuskulatur nicht oder deutlich weniger angespannt werden muss. Halslänge, Halsdicke und Kopfform unterscheiden sich deutlich von Mensch zu Mensch. Eine Nackenstütze sollte daher vorteilhafterweise an die anatomischen Eigenschaften anpassbar bzw. voreinstellbar sein und sich an variable Bewegungsumfänge anpassen können.

Aus dem Stand der Technik ist beispielsweise bekannt, einen pneumatisch aufblasbaren oder textilbasierten gekrümmten Schlauch ("Hörnchen") um den Nacken zu legen. Bekannt sind auch fixe oder höheneinstellbare gewölbte Strukturen, die über eine exoskeletäre Anbindung über Schultern und Rücken an den Körper angebunden sind und im Nackenbereich als Auflage für den Hinterkopf dienen. Eine solche Vorrichtung ist beispielsweise aus der WO 00-78254 A1 bekannt. Eine Schaumstoffauflage auf der Struktur kann den Komfort für den Nutzer erhöhen.

Die Strukturen des Stands der Technik berücksichtigen jedoch die Anatomie und Kopfform des Nutzers nicht individuell.

Die DE 101 96 653 B4 beschreibt eine Kopfstütze mit einem Flügelelement, einem Kopfstützenabschnitt, und einem ersten Gelenkmechanismus, um eine Drehbewegung zwischen dem Flügelelement und dem Kopfstützenabschnitt zu erlauben, wobei das erste Flügelelement in eine erste Vielzahl von Positionen innerhalb eines ersten Bewegungsbereichs und zusätzlich in eine verstaute Position positionierbar ist, in der das erste Flügelelement mit einem Seitenprofil des Kopfstützenabschnitts fluchtet, wobei der erste Gelenkmechanismus innerhalb des ersten Bewegungsbereichs eine ausreichende, dem Gelenkmechanismus immanente Reibung aufweist, um eine Position eines Kopfes, der sich gegen das Flügelelement lehnt, aufrechtzuerhalten.

Aufgabe der vorliegenden Erfindung ist es daher, eine Nackenstütze anzugeben, die sich flexibel an die Kopfform des Nutzers anpassen kann und die vorteilhafterweise auch an andere anatomische Parameter wie beispielsweise die Halslänge des Nutzers anpassbar ist.

Die Aufgabe wird gelöst durch die Nackenstütze nach Anspruch 1. Die abhängigen Ansprüche geben vorteilhafte Weiterbildungen der erfindungsgemäßen Nackenstütze an.

Erfindungsgemäß wird eine Nackenstütze angegeben, die zum einen eine Körperanbindung aufweist, mit der die Nackenstütze am Oberkörper einer Person, dem Nutzer, befestigbar ist, die außerdem eine Kopfstütze und eine Haltevorrichtung aufweist, wobei die Haltevorrichtung an der Körperanbindung angeordnet ist und wobei die Kopfstütze an der Haltevorrichtung angeordnet ist. Die Kopfstütze ist also über die Haltevorrichtung an der Körperanbindung angeordnet.

Unter dem Oberkörper einer Person soll hier vorzugsweise der Bereich des Brustkorbs verstanden werden. Die Körperanbindung kann dann so ausgestaltet sein, das mit ihr die Nackenstütze am Oberkörper der Person befestigbar ist, vorzugsweise auf deren Rücken.

Die Kopfstütze dient hier dazu, den Kopf der Person abzustützen, vorzugsweise nach hinten, d.h. in Richtung des Rückens der Person.

Erfindungsgemäß weist die Kopfstütze zwei Schenkel auf, die elastisch biegbar sind und die durch eine Mehrzahl an Querstreben miteinander verbunden sind. Derartige Strukturen sind auch als Finray-Strukturen oder Fishtail-Strukturen bekannt. Dass die Schenkel elastisch biegbar sind, kann auf unterschiedliche Weise erreicht werden. Zum einen können die Schenkel ein biegeelastisches Material aufweisen oder daraus bestehen. So können die Schenkel beispielsweise aus elastisch biegbarem Kunststoff oder Blech hergestellt sein. Es ist aber auch möglich, die Biegeelastizität der Schenkel dadurch herzustellen, dass starre Elemente über gefederte Drehgelenke miteinander verbunden sind. Die starren Elemente der Schenkel können dann bei Fehlen äußerer Kräfte jeweils in einer Ebene liegen, in der auch die Drehachsen der die Elemente verbindenden Drehgelenke liegen können. Wirkt eine Biegekraft auf den entsprechenden Schenkel, so werden die Elemente um die Drehgelenkte gegen deren Federkraft bewegt. Die Ferderkräfte können eine Rückstellung in die ebene Form bewirken, so dass der entsprechende Schenkel elastisch biegbar ist. Eine derartige Struktur ist beispielsweise in der DE 10 2005 010 380 B4 beschrieben. Bevorzugterweise weisen die Schenkel zumindest eine gerade Kante auf, welches jene Kante sein kann, an der die miteinander verbunden sind. Besonders bevorzugt können die Schenkel jeweils einen rechteckigen Umfang haben oder rechteckig sein. Die Schenkel können beispielsweise als rechteckige Flächen ausgestaltet sein. Die Querstreben können dann an den einander zugewandten Oberflächen der Schenkel angeordnet sein. Bevorzugterweise können die Schenkel eine gemeinsame Ebene aufspannen, zu welcher die Querstreben parallel verlaufen. Die Querstreben sind vorzugsweise gerade und starr. Die Schenkel der Kopfstütze können auch ein rechtwinkliges oder gleichschenkliges Dreieck bilden. Die Länge und Breite der Längsstreben sowie die Anzahl und Breite der Querstreben können variieren und auf diese Weise sich unterschiedlich stark verformende, eine Gegenkraft aufbringende Strukturen bilden. An ihren der Haltevorrichtung zugewandten Kanten können die Schenkel beispielsweise auf einer Platte, z.B. einer kreisförmigen Platte angeordnet sein, z.B. an deren Außenrand.

In einer vorteilhaften Ausgestaltung können die beiden Schenkel nicht-parallel zueinander angeordnet sein. Sie liegen dann an jeweils einer ihrer Kanten aneinander an und schließen an dieser Kante einen Winkel ein, der vorzugsweise größer als 0° und kleiner als 180° ist. Bevorzugterweise ist der Winkel ein spitzer Winkel, also ≤ 90°. Die Querstreben können dann in diesem Winkel verlaufen. Jene Kanten, an denen die Schenkel aneinander anliegen, sind vorzugsweise jener der Haltevorrichtung abgewandten, d.h. am weitesten von der Haltevorrichtung entfernten Kanten.

Die Mehrzahl an Querstreben ist vorzugsweise parallel zueinander angeordnet. Sie können auch parallel verlaufen zu einer Fläche, auf der die Schenkel angeordnet sein können.

Die Querstreben können an den Schenkeln vorteilhafterweise über ein Gelenk angeordnet sein. Dieses Gelenk kann besonders bevorzugt ein Drehgelenk, ein Scharnier oder ein Festkörpergelenk sein. Jeder Schenkel weist also an jedem seiner Enden eines dieser Gelenke auf und ist über dieses Gelenk mit dem jeweiligen Schenkel verbunden.

Die beschriebene Struktur der Kopfstütze passt sich von selbst der Kopfform an, wenn ein Nutzer seinen Kopf auf der Kopfstütze lagert. Auf diese Weise hat die Kopfstütze unabhängig von der Form des Kopfes bei der Benutzung immer die richtige Form.

In einer vorteilhaften Ausgestaltung kann die Kopfstütze mit einem textilen Überzug bedeckt sein. Ein solcher kann beispielsweise vor Schmutzablagerungen schützen und zur Weichheit der Kopfstütze beitragen. Der textile Überzug kann vorteilhaft mit einer sehr reibungsarmen Oberfläche beschichtet sein und dadurch die Bewegung der Kopfhaut und der Haare reibungsarm ermöglichen. Vorteilhafterweise kann der textile Überzug zwei, mehr oder alle Finray-Strukturen überbrücken, sodass Einzelstrukturen optisch kaschiert werden.

In einer vorteilhaften Ausgestaltung der Erfindung kann die Haltevorrichtung mit der Kopfstütze über eine Linearführung verbunden sein. Diese Linearführung kann an einem der beiden Elemente, also an der Haltevorrichtung oder der Kopfstütze, eine Nut aufweisen und an dem anderen dieser Elemente einen Zapfen aufweisen, der in der Nut entlang einer Längsrichtung der Nut verschiebbar ist. Es ist hier bevorzugt, wenn die Kopfstütze den Zapfen aufweist. Vorteilhafterweise kann der Zapfen einen kreisförmigen Querschnitt zu einer Öffnungsfläche der Nut parallelen Querschnittsfläche haben. Eine solche kreisförmige Querschnittsfläche ermöglicht eine Drehbarkeit der Kopfstütze um eine senkrecht auf der Länge der Nut stehenden Achse. Die Nut kann zum Beispiel eine T-Nut-Schiene sein. Sind die Schenkel der Kopfstütze wie oben beschrieben auf einer Fläche oder einem kreisförmigen Teller angeordnet, so kann vorteilhafterweise der Zapfen auf einer den Schenkeln abgewandten Oberfläche der Fläche oder des Tellers in dessen Mitte angeordnet sein.

Vorteilhafterweise weist die Nut entlang ihrer Längsrichtung eine Krümmung auf, deren Krümmungsradius so gemessen ist, dass der Mittelpunkt der Krümmung bei bestimmungsgemäßer Verwendung der Nackenstütze im Kopf der tragenden Person liegt, vorzugsweise in dessen Mittelpunkt. Da die Kopfstütze selbst flexibel ist, kann der Radius auf die Abmessungen einer Durchschnittsperson bemessen werden. Hierdurch lässt sich die Kopfstütze an eine geeignete Position am Kopf verschieben.

In einer vorteilhaften Ausgestaltung kann, wenn die Haltevorrichtung die Nut aufweist, in der Nut eine Mehrzahl an Kopfstützen angeordnet sein. Vorzugsweise sind diese Kopfstützen gleich ausgestaltet, sodass ihre Schenkel eine gemeinsame Auflagefläche für den Kopf aufspannen.

In einer vorteilhaften Ausgestaltung der Erfindung kann die Haltevorrichtung an der Körperanbindung über eine Schiene angeordnet sein, auf der die Haltevorrichtung gegenüber der Körperanbindung verschiebbar ist. Vorteilhafterweise ist die Schiene so orientiert, dass die Verschiebung in Richtung der Kopfstütze erfolgt. Beispielsweise kann die Schiene im Wesentlichen parallel zur Wirbelsäule der tragenden Person verlaufen. Optional können Befestigungsmechanismen vorgesehen sein, mit denen eine Verschiebung der Haltevorrichtung auf der Schiene blockierbar ist. Beispielsweise können hierzu Schrauben vorgesehen sein, die durch die Haltevorrichtung geschraubt sind und im eingeschraubten Zustand auf die Schiene der Körperanbindung drücken. Eine solche Schiene erlaubt eine individuelle Anpassung der Nackenstütze an die Länge des Halses des Benutzers.

In einer bevorzugten Ausgestaltung der Erfindung kann die Haltevorrichtung drei Teile aufweisen, zum einen ein Kopfstützenhalteteil, an dem die Kopfstütze angeordnet ist, außerdem ein Körperanbindungsteil, mit dem die Haltevorrichtung an der Körperanbindung angeordnet ist sowie ein Verbindungsteil, über das das Kopfstützenhalteteil und das Körperanbindungsteil miteinander verbunden sind.

In dieser Ausgestaltung ist es bevorzugt, wenn das Kopfstützenhalteteil mit dem Verbindungsteil über ein erstes Drehgelenk verbunden ist, das um eine Achse drehbar ist, die senkrecht steht zu einer die Kopfstütze und die Haltevorrichtung verbindenden Linie oder zu einer Längsrichtung des Verbindungsteils, und die parallel liegt zu einer Rückenfläche der Körperanbindung, die im getragenen Zustand auf dem Rücken der Person anlegbar ist oder anliegt oder die parallel liegt zum Rücken der tragenden Person im bestimmungsgemäß getragenen Zustand. Ein solches Drehgelenk erlaubt die Anpassung des Winkels der Kopfstütze an den Hinterkopf der sie tragenden Person.

In einer weiteren vorteilhaften Ausgestaltung kann der Verbindungsteil mit dem Körperanbindungsteil über ein zweites Drehgelenk verbunden sein, das um eine Achse drehbar ist, die senkrecht steht zu einer die Kopfstütze und die Haltevorrichtung verbindenden Linie oder senkrecht steht zu einer Längsrichtung des Verbindungsteils, und die parallel liegt zu einer Rückenfläche der Körperanbindung, die im getragenen Zustand auf dem Rücken der Person anlegbar ist oder anliegt oder die parallel liegt zum Rücken der Person im bestimmungsgemäß getragenen Zustand. Ein solches Gelenk erlaubt eine Anpassung der Nackenstütze an den Verlauf des Halses der tragenden Person.

Vorteilhafterweise weisen beide genannten Gelenke gegebenenfalls Mechanismen auf, mit denen die Bewegung der Gelenke blockierbar ist. Insbesondere bei dem den Verbindungsteil mit dem Körperanbindungsteil verbindenden Gelenk kann auch ein Schnellspanner zum Blockieren dieses Gelenks vorteilhaft zum Einsatz kommen.

In einer bevorzugten Ausgestaltung der Erfindung kann das Verbindungsteil eine Linearführung aufweisen, über die das Kopfstützenhalteteil und das Körperanbindungsteil gegeneinander beweglich gelagert sind. Jene Richtung, in der die Linearführung beweglich ist, kann als Längsrichtung des Verbindungsteils angesehen werden. Bevorzugterweise kann dann das Verbindungsteil außerdem eine Feder aufweisen, mit der die Bewegung der Linearführung des Verbindungsteils abgefedert werden kann. Bevorzugterweise kann diese Feder eine Druckfeder sein. Die Linearführung kann beispielsweise über ein oder zwei Paare ineinander verlaufender koaxialer Zylinder realisiert sein. Die genannte Druckfeder kann dann im Inneren der Zylinder verlaufen und sich an Stirnflächen der jeweiligen Zylinder abstützen. Durch eine solche gefederte Linearführung ist es möglich, dass die Nackenstütze die Bewegung des Kopfes nach hinten abfedert.

In einer vorteilhaften Ausgestaltung der Erfindung kann die Körperanbindung eine Rückenfläche aufweisen, mit der sie im getragenen Zustand auf dem Rücken der Person anlegbar ist oder anliegt. Darüber hinaus kann die Körperanbindung vorteilhafterweise ein Tragegeschirr aufweisen, mit dem die Rückenfläche am Oberkörper der Person befestigbar ist. Ein solches Tragegeschirr kann beispielsweise Gurte aufweisen, von denen jeweils einer über die Schultern und unter dem entsprechenden Arm vom Körperanbindungsteil zum Körperanbindungsteil zurückverläuft und von denen einer beispielsweise um den Brustkorb herum verläuft.

Die erfindungsgemäße Nackenstütze ermöglicht durch die Körperanbindung eine leichte und bequeme Tragweise. Die Körperanbindung hält die Nackenstütze stabil im Nackenbereich. Die verschiedenen Einstellmechanismen erlauben eine Feinpositionierung der Nackenstütze, eine Voranpassung an einen individuellen Kopf-Nacken-Abstand, das Dämpfen und Überstoppen von diagonal nach hinten gerichteten Kopfbewegungen sowie die radiale Ausrichtung der Kopfstütze zum Kopf hin oder die sagittale Ausrichtung zum Körper hin oder eine Stellung dazwischen. Die erfindungsgemäße selbstadaptive Nackenstütze erlaubt die Anpassung an jegliche konvexe Kopfformen und stützt den Kopf ab, sodass die Nackenmuskulatur nur wenig angespannt werden muss, um den Kopf in Position zu halten.

Im Folgenden soll die Erfindung anhand einiger Figuren beispielhaft erläutert werden. Gleiche Bezugszeichen kennzeichnen dabei gleiche oder entsprechende Merkmale. Die in den Beispielen beschriebenen Merkmale können auch unabhängig vom entsprechenden Beispiel realisiert sein und zwischen unterschiedlichen Beispielen kombiniert werden.

Es zeigt
- Figur 1: eine erste mögliche Ausgestaltung einer erfindungsgemäßen Nackenstütze,
- Figur 2: eine zweite Ausgestaltung einer erfindungsgemäßen Nackenstütze und
- Figur 3: eine mögliche Ausgestaltung einer Kopfstütze zur Verwendung in der erfindungsgemäßen Nackenstütze.

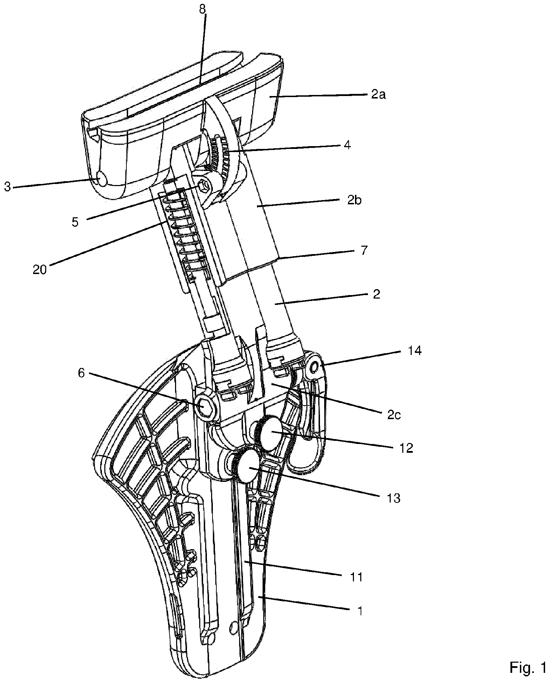

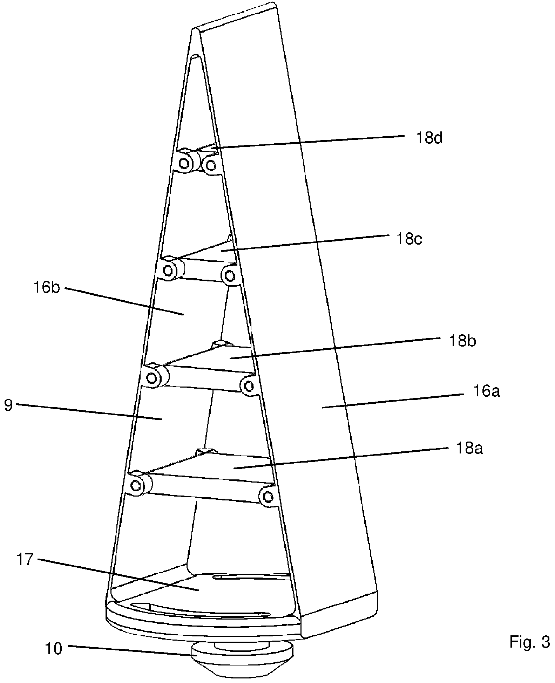

Figur 1 zeigt ein erstes Beispiel einer erfindungsgemäßen Nackenstütze, wobei zur Übersichtlichkeit eine Kopfstütze 9 nicht gezeigt ist. Die in Figur 1 gezeigte Vorrichtung kann mit der Kopfstütze 9 wie beispielsweise in Figur 3 kombiniert werden. Die in Figur 1 gezeigte Nackenstütze weist eine Körperanbindung 1 auf, die am Oberkörper einer Person befestigbar ist, beispielsweise mittels Riemen, die in der Figur nicht gezeigt sind. An der Körperanbindung 1 ist eine Haltevorrichtung 2 angeordnet, die zum einen einen Kopfstützenhalteteil 2a, ein Verbindungsteil 2b sowie ein Körperanbindungsteil 2c enthält. An dem Kopfstützenhalteteil 2a ist die Kopfstütze 9 anordenbar, wie sie beispielsweise in Figur 3 gezeigt ist. Das Verbindungsteil 2b verbindet das Kopfstützenhalteteil 2a und das Körperanbindungsteil 2c. Im in Fig. 1 gezeigten Beispiel ist das Kopfstützenhalteteil 2a mit dem Verbindungsteil 2b über ein erstes Drehgelenk 3 verbunden. Das Drehgelenk 3 ist um eine Achse drehbar, die senkrecht steht zu einer die Kopfstütze 9 und die Körperanbindung 1 verbindenden Linie und die parallel liegt zur Rückenfläche der Körperanbindung 1. Im gezeigten Beispiel ist das Gelenk 3 am Kopfstützenhalteteil 2a umgeben von einem geriffelten Bogen 4, in den ein stoppendes Element durch senkrecht auf dem geriffelten Teil 4 stehende Öffnungen 5 eingreifen kann, um den geriffelten Bogen 4 zu arretieren und damit die Bewegung des Gelenks 3 zu blockieren. Auf diese Weise kann die Kopfstütze 9 in einer festen Position arretiert werden.

Ein weiteres Gelenk 6, dessen Gelenkachse parallel liegt zu der Gelenkachse des Gelenks 3, also senkrecht zu einer die Kopfstütze 9 und die Körperanbindung 1 verbindenden Linie und parallel zur Rückenplatte der Körperanbindung 1, ist zwischen dem Verbindungsteil 2b und dem Körperanbindungsteil 2c vorgesehen. Über dieses Gelenk kann die Nackenstütze an den Verlauf des Halses der tragenden Person angepasst werden.

Das Verbindungsteil 2b weist im in Figur 1 gezeigten Beispiel eine Linearführung 7 auf, durch Verschieben von welcher die Länge des Verbindungsteils 2b, also der Abstand zwischen dem Kopfstützenhalteteil 2a und dem Körperanbindungsteil 2c veränderbar ist. Die Linearführung 7 kann in ihrem Inneren Federn, vorzugsweise Druckfedern 20 aufweisen, von denen in Figur 1 eine im aufgeschnittenen vorderen Zylinder zu erkennen ist. Auf diese Weise kann die Verschiebung der Linearführung 7 gefedert sein und die Linearführung 7 so zur Abfederung des Kopfes nach hinten beitragen. Im gezeigten Beispiel weist die Linearführung 7 eine Kombination von jeweils zwei ineinander verlaufenden Zylindern auf, die bei Verschiebung der Linearführung 7 ineinander geschoben werden. In Figur 1 ist der vordere Zylinder aufgeschnitten, um die innere Struktur zu zeigen. Vorteilhafterweise sind beide Zylinder geschlossen.

Das Kopfstützenhalteteil 2a weist auf seiner dem Verbindungsteil 2b abgewandten Seite eine Linearführung 8 auf, die im getragenen Zustand um den Kopf der Person horizontal verläuft. Die Linearführung 8 ist hierzu gebogen, um grob der Form des Kopfes zu folgen. Wie in Figur 3 gezeigt ist, weist die Kopfstütze 9 einen Zapfen 10 auf, der in der Linearführung 8 verschiebbar ist. Im in Figur 1 gezeigten Beispiel ist die Linearführung 8 als T-Nut-Schiene ausgebildet. Die Kopfstütze 9 ist daher entlang der Nut verschiebbar. Vorteilhaft kann der Zapfen 10 an der Kopfstütze 9 einen kreisförmigen Querschnitt in einer Ebene parallel zur Öffnungsebene der Nut 8 aufweisen, wodurch die Kopfstütze um eine parallel zum Zapfen 10 verlaufende Achse drehbar ist.

In Figur 1 ist die Haltevorrichtung 2 an der Körperanbindung 1 über eine Schiene 11 angeordnet, entlang derer das Körperanbindungsteil 2c verschiebbar ist. Die Schiene 11 verläuft hier im Wesentlichen in einer Richtung parallel zur auf die Kopfstütze 9 gerichteten Richtung. Mittels einer Schraube 13, die durch das Körperanbindungsteil 2c auf die Schiene 11 drückt, kann die Verschiebung entlang der Schiene 11 arretiert werden.

Im in Figur 1 gezeigten Beispiel weist das Körperanbindungsteil 2c außerdem eine Schraube 12 auf, mit der ein Winkel des Gelenks 6 einstellbar ist. Mittels eines Schnellspanners 14 ist die Drehung um das Gelenk 6 arretierbar.

Die Körperanbindung 1 hat im gezeigten Beispiel einen im Wesentlichen dreieckigen Umfang, wobei jene der Kopfstütze zugewandte Kante konvex gekrümmt ist und die beiden seitlichen Kanten konkav gekrümmt sind. Auf diese Weise liegt die Körperanbindung 1 besonders bequem auf dem Rücken der tragenden Person. Die Körperanbindung 1 ist gerippt, um große Stabilität bei geringem Gewicht zu ermöglichen.

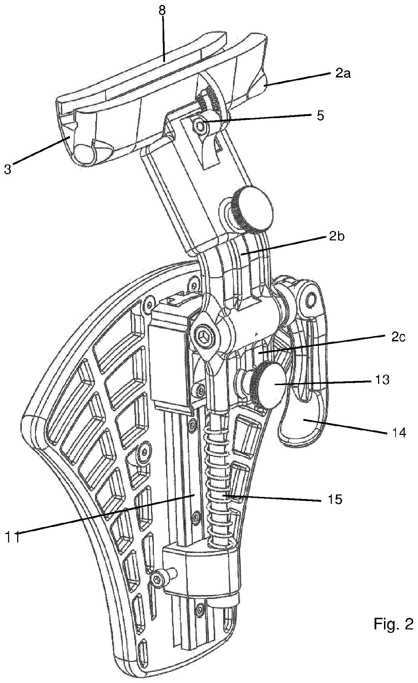

Figur 2 zeigt eine alternative Ausgestaltung einer erfindungsgemäßen Nackenstütze, wobei wiederum die Kopfstütze 9 nicht montiert ist. Wie in Figur 1 kann jedoch eine wie in Figur 3 gezeigte Kopfstütze 9 vorgesehen werden.

Die Körperanbindung 1 und das Kopfstützenhalteteil 2a sind im Wesentlichen wie in Figur 1 gezeigt ausgestaltet. Wie in Figur 1 weisen sie ein Gelenk 3, eine Nut 8 am Kopfstützenhalteteil 2a und eine Körperanbindung 1 auf, zu deren Beschreibung auf Figur 1 verwiesen werden soll. Auch das Gelenk 6 ist in Figur 2 wie in Figur 1 gezeigt ausgestaltet.

Anders als in Figur 1 ist jedoch das Verbindungsteil 2b nicht mit einer Linearführung 7 ausgestattet. Andererseits ist jedoch die Beweglichkeit des Körperanbindungsteils 2c auf der Schiene 11 der Körperanbindung 1 hier mittels einer Feder 15 abgefedert. Es entfällt hier die Arretierungsschraube 13 der Figur 1, sodass der Kopf bei Bewegungen nach hinten über die Feder 15 abfederbar ist. Vorteilhafterweise ist die Feder 15 durch eine hier nicht gezeigte Abdeckung bedeckt.

Figur 3 zeigt beispielhaft eine Kopfstütze 9, wie sie an den in den Figuren 1 und 2 gezeigten Kopfstützenhalteteilen 2a anordenbar ist. Die Kopfstütze 9 weist zwei Schenkel 16a und 16b auf, die im gezeigten Beispiel als rechteckige Flächen ausgestaltet sind. Die Schenkel 16a und 16b sind biegeelastisch und mit einer Kante auf einer Tragefläche 17 angeordnet, die im gezeigten Beispiel kreisförmig ist und in deren Mitte der Zapfen 10 auf jener den Schenkeln 16a, 16b abgewandten Seite angeordnet ist. Die Schenkel 16a und 16b sind an ihren dem Tragteller 17 abgewandten Kanten miteinander verbunden. Die Schenkel 16a und 16b sind darüber hinaus über eine Mehrzahl an Streben 18a, 18b, 18c, 18d über jeweils ein Gelenk, beispielsweise ein Drehgelenk, mit den entsprechenden Schenkeln 16a, 16b verbunden. Dabei sind die Streben miteinander verbunden. Die Struktur aus biegeelastischen Schenkeln 16a, 16b und Querstreben 18, 18b, 18c, 18d ist auch als Finray-Struktur bekannt.

## Patentansprüche

1. Nackenstütze mit
einer Körperanbindung (1), die am Oberkörper einer Person befestigbar ist,
einer Kopfstütze (9) und
einer Haltevorrichtung (2), wobei die Haltevorrichtung (2) an der Körperanbindung (1) angeordnet ist und wobei die Kopfstütze (9) an der Haltevorrichtung (2) angeordnet ist,
**dadurch gekennzeichnet, dass**
die Kopfstütze (9) zwei Schenkel (16a, 16b) aufweist, die elastisch biegbar sind und die durch eine Mehrzahl an Querstreben (18a, 18b, 18c, 18d) miteinander verbunden sind.

2. Nackenstütze nach dem vorhergehenden Anspruch, wobei die zwei Schenkel (16a, 16b) nicht parallel zueinander aneinander angeordnet sind und/oder einen Winkel größer als Null und kleiner als 180°, vorzugsweise kleiner oder gleich 90° einschließend aneinander angeordnet sind.

3. Nackenstütze nach einem der vorhergehenden Ansprüche, wobei die zwei Schenkel (16a, 16b) an ihrem jeweiligen von der Haltevorrichtung (2) am weitesten entfernten Ende aneinander angeordnet sind.

4. Nackenstütze nach einem der vorhergehenden Ansprüche, wobei die Schenkel (16a, 16b) jeweils einen rechteckigen Umfang haben oder rechteckig sind.

5. Nackenstütze nach einem der vorhergehenden Ansprüche, wobei die Mehrzahl an Querstreben (18a, 18b, 18c, 18d) parallel zueinander angeordnet sind.

6. Nackenstütze nach einem der vorhergehenden Ansprüche, wobei die Querstreben (18a, 18b, 18c, 18d) jeweils über ein Drehgelenk, Scharnier oder Festkörpergelenk an den Schenkeln befestigt sind.

7. Nackenstütze nach einem der vorhergehenden Ansprüche, wobei die Kopfstütze (9) mit einem textilen Überzug bedeckt ist.

8. Nackenstütze nach einem der vorhergehenden Ansprüche, wobei die Haltevorrichtung (2) mit der Kopfstütze (9) über eine Linearführung (8) verbunden ist, wobei die Linearführung (8) an einem aus Haltevorrichtung (2) und Kopfstütze (9) eine Nut (8) aufweist und an dem anderen aus Haltevorrichtung und Kopfstütze einen Zapfen (10) aufweist, der in der Nut entlang einer Längsrichtung der Nut (8) verschiebbar ist, wobei vorzugsweise der Zapfen (10) einen kreisförmigen Querschnitt in einer zu einer Öffnungsfläche der Nut (8) parallelen Querschnittsfläche hat.

9. Nackenstütze nach einem der vorhergehenden Ansprüche, wobei die Haltevorrichtung (2) an der Körperanbindung über eine Schiene (11) angeordnet ist, auf der die Haltevorrichtung (2) gegenüber der Körperanbindung (1) in Richtung der Kopfstütze (9) verschiebbar ist.

10. Nackenstütze nach einem der vorhergehenden Ansprüche, wobei die Haltevorrichtung (2) ein Kopfstützenhalteteil (2a) aufweist, an dem die Kopfstütze (9) angeordnet ist, ein Körperanbindungsteil (2c) aufweist, mit dem sie an der Körperanbindung (1) angeordnet ist, und ein Verbindungsteil (2b) aufweist, über den das Kopfstützenhalteteil (2a) und das Körperanbindungsteil (2c) miteinander verbunden sind.

11. Nackenstütze nach dem vorhergehenden Anspruch, wobei das Kopfstützenhalteteil (2a) mit dem Verbindungsteil (2b) über ein erstes Drehgelenk (3) verbunden ist, das um eine Achse drehbar ist, die senkrecht steht zu einer die Kopfstütze (9) und die Haltevorrichtung (2) verbindenden Linie und parallel liegt zu einer Rückenfläche der Körperanbindung (1), die im getragenen Zustand auf dem Rücken der Person anlegbar ist oder anliegt.

12. Nackenstütze nach einem der beiden vorhergehenden Ansprüche, wobei das Verbindungsteil (2b) mit dem Körperanbindungsteil (2c) über ein Drehgelenk (6) verbunden ist, das um eine Achse drehbar ist, die senkrecht steht zu einer die Kopfstütze (9) und die Haltevorrichtung (2) verbindenden Linie und parallel liegt zu einer Rückenfläche der Körperanbindung (1), die im getragenen Zustand auf dem Rücken der Person anlegbar ist oder anliegt.

13. Nackenstütze nach einem der Ansprüche 10 bis 12, wobei der Verbindungsteil (2b) eine Linearführung (7) aufweist, über die das Kopfstützenhalteteil (2a) und das Körperanbindungsteil (2c) gegeneinander beweglich gelagert sind, wobei das Verbindungsteil (2b) außerdem eine Feder (20) aufweist, mit der eine Bewegung der Linearführung des Verbindungsteils abgefedert ist, wobei vorzugsweise die Feder (20) eine Druckfeder ist.

14. Nackenstütze nach einem der vorhergehenden Ansprüche, wobei die Körperanbindung eine Rückenfläche aufweist, mit der die Körperanbindung (1) im getragenen Zustand auf dem Rücken der Person anlegbar ist oder anliegt, und außerdem ein Tragegeschirr aufweist, mit dem die Rückenfläche am Oberkörper der Person befestigbar ist.

## Claims

1. A neck support comprising
a body connection (1) that can be attached to a person's upper body,
a headrest (9) and
a holding device (2), wherein the holding device (2) is arranged on the body connection (1) and wherein the headrest (9) is arranged on the holding device (2),
**characterized in that**
the headrest (9) has two legs (16a, 16b) which are elastically bendable and which are connected to one another by a plurality of cross struts (18a, 18b, 18c, 18d).

2. The neck support according to the preceding claim, wherein the two legs (16a, 16b) are not arranged parallel to each other and/or are arranged together forming an angle greater than zero and less than 180°, preferably less than or equal to 90°.

3. The neck support according to one of the preceding claims, wherein the two legs (16a, 16b) are arranged together at their respective end which is furthest from the holding device (2).

4. The neck support according to one of the preceding claims, wherein the legs (16a, 16b) each have a rectangular circumference or are rectangular.

5. The neck support according to one of the preceding claims, wherein the plurality of cross struts (18a, 18b, 18c, 18d) are arranged parallel to each other.

6. The neck support according to one of the preceding claims, wherein the cross struts (18a, 18b, 18c, 18d) are each attached to the legs via a pivot joint, hinge or solid state joint.

7. The neck support according to one of the preceding claims, wherein the headrest (9) is covered with a textile cover.

8. The neck support according to one of the preceding claims, wherein the holding device (2) is connected to the headrest (9) via a linear guide (8), wherein the linear guide (8) has a groove (8) on one of the holding device (2) and the headrest (9) and has a pin (10) on the other of the holding device and the headrest, which pin is displaceable in the groove along a longitudinal direction of the groove (8), the pin (10) preferably having a circular cross-section in a cross-sectional area parallel to an opening area of the groove (8).

9. The neck support according to one of the preceding claims, wherein the holding device (2) is arranged on the body connection via a rail (11), on which the holding device (2) is displaceable relative to the body connection (1) in the direction of the headrest (9).

10. The neck support according to one of the preceding claims, wherein the holding device (2) comprises a headrest holding part (2a) on which the headrest (9) is arranged, a body connection part (2c) with which it is arranged on the body attachment (1), and a connecting part (2b) via which the headrest holding part (2a) and the body attachment part (2c) are connected to one another.

11. The neck support according to the preceding claim, wherein the headrest holding part (2a) is connected to the connecting part (2b) via a first pivot joint (3) which is rotatable about an axis which is perpendicular to a line connecting the headrest (9) and the holding device (2) and is parallel to a back surface of the body connection (1) which, in the worn state, can be placed against or rests against the person's back.

12. The neck support according to one of the two preceding claims,
wherein the connecting part (2b) is connected to the body attachment part (2c) via a pivot joint (6) which is rotatable about an axis which is perpendicular to a line connecting the headrest (9) and the holding device (2) and lies parallel to a back surface of the body connection (1) which, in the worn state, can be placed against or rests against the person's back.

13. The neck support according to one of claims 10 to 12, wherein the connecting part (2b) has a linear guide (7), via which the headrest holding part (2a) and the body connecting part (2c) are mounted movably relative to one another, wherein the connecting part (2b) also has a spring (20), with which a movement of the linear guide of the connecting part is cushioned, wherein preferably the spring (20) is a compression spring.

14. The neck support according to one of the preceding claims, wherein the body connection has a back surface with which the body connection (1) can be placed or rests on the person's back in the worn state, and also has a carrying harness with which the back surface can be fastened to the person's upper body.

## Revendications

1. Appuie-nuque avec
une fixation corporelle (1) qui peut être fixée avec le haut du corps d'une personne,
un appuie-tête (9) et
un dispositif de maintien (2), dans lequel le dispositif de maintien (2) est disposé sur la fixation corporelle (1) et dans lequel l'appuie-tête (9) est disposé sur le dispositif de maintien (2),
**caractérisé en ce que**
l'appuie-tête (9) comprend deux branches (16a, 16b) qui sont flexibles de manière élastique et qui sont reliées entre elles à l'aide d'une pluralité d'entretoises transversales (18a, 18b, 18c, 18d).

2. Appuie-nuque selon la revendication précédente, dans lequel les deux branches (16a, 16b) ne sont pas disposées parallèlement entre elles et/ou sont disposées de façon à former entre elles un angle supérieur ou égal à zéro et inférieur à 180°, de préférence inférieur ou égal à 90°.

3. Appuie-nuque selon l'une des revendications précédentes, dans lequel les deux branches (16a, 16b) sont disposées l'une par rapport à l'autre au niveau leur extrémité respective la plus éloignée du dispositif de maintien (2).

4. Appuie-nuque selon l'une des revendications précédentes, dans lequel les deux branches (16a, 16b) présentent un contour rectangulaire ou sont rectangulaires.

5. Appuie-nuque selon l'une des revendications précédentes, dans lequel la pluralité d'entretoises transversales (18a, 18b, 18c, 18d) sont disposées parallèlement entre elles.

6. Appuie-nuque selon l'une des revendications précédentes, dans lequel les entretoises transversales (18a, 18b, 18c, 18d) sont fixées aux branches chacune à l'aide d'une articulation rotative, d'une charnière ou d'une articulation à corps fixe.

7. Appuie-nuque selon l'une des revendications précédentes, dans lequel l'appuie-tête (9) est recouvert d'une housse textile.

8. Appuie-nuque selon l'une des revendications précédentes, dans lequel le dispositif de maintien (2) est relié avec l'appuie-tête (9) par l'intermédiaire d'un guidage linéaire (8), dans lequel la rainure (8) comprend, au niveau d'un élément constitué d'un dispositif de maintien (2) et de l'appuie-tête (9), une rainure (8) et comprend, au niveau de l'autre élément constitué d'un dispositif de maintien et de l'appuie-tête, un axe (10) qui peut coulisser dans la rainure le long d'une direction longitudinale de la rainure (8), dans lequel, de préférence, l'axe (10) présente une section transversale circulaire dans une surface de section transversale parallèle à la surface d'ouverture de la rainure (8).

9. Appuie-nuque selon l'une des revendications précédentes, dans lequel le dispositif de maintien (2) est disposé sur la fixation corporelle par l'intermédiaire d'un rail (11) sur lequel le dispositif de maintien (2) peut coulisser par rapport à la fixation corporelle (1) en direction de l'appuie-tête (9).

10. Appuie-nuque selon l'une des revendications précédentes, dans lequel le dispositif de maintien (2) comprend une partie de maintien d'appuie-tête (2a) sur laquelle l'appuie-tête (9) est disposé, une partie de fixation corporelle (2c), avec laquelle il est fixé à la fixation corporelle (1) et une partie de liaison (2b) par l'intermédiaire de laquelle la partie de maintien d'appuie-tête (2a) et la partie de fixation corporelle (2c) sont reliées entre elles.

11. Appuie-nuque selon la revendication précédente, dans lequel la partie de maintien d'appuie-tête (2a) est reliée avec la partie de fixation corporelle (2b) par l'intermédiaire d'une première articulation rotative (3) qui peut tourner autour d'un axe qui est perpendiculaire à une ligne reliant l'appuie-tête (9) et le dispositif de maintien (2) et qui est parallèle à une surface dorsale de la fixation corporelle (1), qui peut être appuyée ou qui peut s'appuyer, lorsqu'elle est portée, sur le dos de la personne.

12. Appuie-nuque selon l'une des revendications précédentes, dans lequel la partie de liaison (2b) est reliée avec la partie de fixation corporelle (2c) par l'intermédiaire d'une articulation rotative (6) qui peut tourner autour d'un axe qui est perpendiculaire à une ligne reliant l'appuie-tête (9) et le dispositif de maintien (2) et qui est parallèle à une surface dorsale de la fixation corporelle (1), qui peut être appuyée ou qui peut s'appuyer, lorsqu'elle est portée, sur le dos de la personne.

13. Appuie-nuque selon l'une des revendications 10 à 12, dans lequel la partie de liaison (2b) comprend un guidage linéaire (7) par l'intermédiaire duquel la partie de maintien d'appuie-tête (2a) et la partie de fixation corporelle (2c) sont logées de manière mobile l'une par rapport à l'autre, dans lequel la partie de liaison (2b) comprend en outre un ressort (20) avec lequel un déplacement du guidage linéaire de la partie de liaison est amorti, dans lequel, de préférence, le ressort (20) est un ressort de compression.

14. Appuie-nuque selon l'une des revendications précédentes, dans lequel la fixation corporelle comprend une surface dorsale avec laquelle la fixation corporelle (1) peut s'appuyer ou est appuyée, lorsqu'elle est portée, sur le dos de la personne et comprend en outre un harnais avec lequel la surface dorsale peut être fixée sur le haut du corps de la personne.
